(19) Europäisches Patentamt
European Patent Office
Office européen des brevets

(11) EP 0 954 321 B1

(12) **EUROPEAN PATENT SPECIFICATION**

(45) Date of publication and mention of the grant of the patent:
**11.01.2006 Bulletin 2006/02**

(21) Application number: **97910157.3**

(22) Date of filing: **04.11.1997**

(51) Int Cl.:
***A61K 31/70*** *(2006.01)* ***A61K 31/28*** *(2006.01)*
***A61K 31/57*** *(2006.01)* ***A61K 31/58*** *(2006.01)*
*A61K 31/57* *(2006.01)* *A61K 31/28* *(2006.01)*

(86) International application number:
**PCT/AU1997/000747**

(87) International publication number:
**WO 1998/019683 (14.05.1998 Gazette 1998/19)**

(54) **SYNERGISTIC COMPOSITIONS CONTAINING GOLD AND A CORTICOSTEROID**

SYNERGISTISCHE, GOLD UND EIN CORTICOSTEROID ENTHALTENDE ZUSAMMENSETZUNGEN.

COMPOSITIONS SYNERGIQUES CONTENANT DE L'OR ET UN CORTICOSTEROIDE

(84) Designated Contracting States:
**AT BE CH DE DK ES FR GB GR IE IT LI PT**

(30) Priority: **04.11.1996 AU PO347396**

(43) Date of publication of application:
**10.11.1999 Bulletin 1999/45**

(73) Proprietor: **Psiron Ltd**
**Macquarie Park NSW 2113 (AU)**

(72) Inventor: **THOMAS, Richard, Edward**
**Killara, NSW 2071 (AU)**

(74) Representative: **Lee, Nicholas John et al**
**Kilburn & Strode,**
**20 Red Lion Street**
**London WC1R 4PJ (GB)**

(56) References cited:
**AU-A- 1 545 688** **AU-A- 3 435 189**

- **THE JOURNAL OF RHEUMATOLOGY, Volume 21, No. 3, issued March 1994, (Toronto, Canada), M. HEYTMAN et al., "The Longterm Effect of Pulsed Corticosteroids on the Efficacy and Toxicity of Chrysotherapy in Rheumatoid Arthritis", pages 435-441, XP002946491**
- **JOURNAL OF THE AMERICAN VETERINARY MEDICAL ASSOCIATION, Volume 186, No. 1, 1 January 1995, U.S., LHRKE P. et al., "Pemphigus Foloaceus in Dogs: A Review of 37 Cases", pages 59-66, XP002946493**
- **JOURNAL OF THE AMERICAN ACADEMY OF DERMATOLOGY, Volume 16, No. 4, April 1987, U.S., THOMAS I. et al., "Gold Therapy and Its Indications in Dermatology", pages 845-854, XP002946492**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 954 321 B1

Printed by Jouve, 75001 PARIS (FR)

## Description

### TECHNICAL FIELD

The present invention relates to pharmaceutical compositions comprising a gold compound in combination with a corticosteroid and their use in the treatment of dermatological disorders.

### BACKGROUND OF THE INVENTION

The effectiveness of gold compounds in the treatment of rheumatoid arthritis has been known since the 1960s. More recently, gold complexes have been employed as therapeutic agents in the treatment of rheumatoid arthritis but their exact mechanism is still unknown. The most commonly used complexes have been water soluble, parenterally administered gold (Au(1)) thiolates such as aurothiomalate (Myocrisin®) and aurothioglucose (Solganol®). Subsequently, a number of alkylphosphine gold complexes displayed anti-arthritic activity when administered orally to adjuvant arthritic rats. Auranofin (1-thio-β-D-glucopyranose 2,3,4.6-tetraacetato-S)-(triethylphosphine)-Au(I)) was among the most potent and efficacious of the compounds tested and is now used in the treatment of rheumatoid arthritis in man.

Gold compounds have also been administered by intravenous and oral routes for the treatment of asthma, tuberculosis, pemphigus vulgaris, various forms of arthritis, cancer and infection. However, treatment with gold compounds has been frequently associated with unacceptable and on occasions serious side effects.

Corticosteroids have also found similar therapeutic applications. The success of topical corticosteroids in the therapy of inflammatory and proliferative disorders of the skin has led to vigorous development of new corticosteroids since their first topical use. An increase in potency has been achieved by chemical modification of the natural corticosteroid, hydrocortisone, without precise knowledge of the mechanism of action of corticosteroids. The development of more potent corticosteroids has extended their usefulness in a wide variety of skin diseases, but, especially with long term use, has led to unwanted effects. Systemic effects such as hypothalmic pituitary-adrenal-axis depression were already known from the systemic use of corticosteroids. Local side effects after topical application were observed only with the more potent synthetic steroids.

The most common serious side effects of topical corticosteroids are thinning of the skin, striae and telangiectasia. During long term treatment with very potent corticosteroids, inflammatory cells are affected and the proliferation of keratinocytes and the activity of fibroblasts are also inhibited.

Fibroblasts synthesize important structural and functional components of the dermis, namely collagen, elastin and glycosaminoglycans. The inhibition of keratinocyte proliferation leads to thinning of the epidermis. Although the effect on the epidermis is usually reversible, the dermis can be irreversibly damaged.

Recently, topical formulations of gold organic complexes have found use in the treatment of skin disorders such as psoriasis. Thus, Australian Patent No. 616,755, describes the use of a topical formulation of auranofin in combination with a corticosteroid in the treatment of local inflammatory conditions such as those associated with psoriasis. In particular, treatment with a formulation comprising auranofin and betamethasone dipropionate demonstrated a remarkable synergy of action when compared to same concentrations of individual active ingredients. The finding that a gold compound can synergise with a corticosteroid enabled the use of considerably lower levels of both the gold compound and a corticosteroid in the formulations, thus enabling more effective therapy while obviating the well known side effects associated with the use of either gold compounds or corticosteroids alone.

Dermatological disorders are frequently associated with manifestations other than just inflammation. For example, psoriasis also contains a component of cellular hyperproliferation (hyperplasia), the mechanism of which is fundamentally different from that of inflammation and thus may not necessarily be affected by the topical gold/corticosteroid formulations. Furthermore, the inflammatory component of different dermatological conditions may range from very mild to very severe, necessitating variations in the formulation, in particular the choice of corticosteroid which would enable not only effective and appropriate treatment of the inflammatory component, but also provide the differential action in dermatological conditions where there is an additional component such as cellular hyperproliferation.

Other immune, autoimmune and infection disorders can also be associated with multiple manifestations, where effective treatment may rely on targeting only one of the manifestations of the disorder, or more than one, depending on the disorder treated and the assessment of the patient.

The present invention is based on a surprising finding that important differences exist between corticosteroids with respect to the degree of potentiation of effects and the type of effect potentiated, when combined with a gold compound. That is to say, different corticosteroids, when combined with a gold compound, do not all have the expected similarity of synergistic action against inflammation and also demonstrate differential synergistic action with respect to inflammation and hyperplasia. In the compositions of the present invention certain corticosteroids synergise with the gold compound to provide a greater effect on the inflammatory component of a disorder, such as psoriasis, while other corticosteroids give rise to compositions with preferential effects on cellular hyperproliferation. It is contemplated that the compositions of the present invention could be effectively used also for the treatment of a variety of systemic, tissue-specific or localised immune, autoimmune and inflammatory disorders.

## SUMMARY OF THE INVENTION

According to a first aspect, the present invention provides a pharmaceutical composition comprising a gold compound and a corticosteroid selected from fluocinolone acetonide and mometasone furoate in combination with a pharmaceutically acceptable carrier, excipient, adjuvant or solvent.

The composition of the first aspect may be formulated for systemic administration, oral administration, local administration, or topical administration or for administration by intra-articular injection.

According to a second aspect, the present invention provides products containing a corticosteroid selected from fluocinolone acetonide and hydrocortisone, and a gold compound as a combined preparation for simultaneous, separate or sequential use in reducing epidermal hyperplasia. The epidermal hyperplasia may be associated with psoriasis.

According to a third aspect, the present invention provides products containing a corticosteroid selected from fluocinolone acetonide and mometasone furoate, and a gold compound as a combined preparation for simultaneous, separate or sequential use in reducing inflammation.

In the third aspect, the inflammation may be reduced in a skin condition selected from the group consisting of atopic dermatitis, contact dermatitis, seborrhoeic dermatitis and psoriasis.

According to a fourth aspect, the present invention provides the use of (i) a corticosteroid selected from fluocinolone acetonide and hydrocortisone, and (ii) a gold compound in the manufacture of a medicament for reducing epidermal hyperplasia. The epidermal hyperplasia may be associated with psoriasis.

According to a fifth aspect, the present invention provides the use of (i) a corticosteroid selected from fluocinolone acetonide and mometasone furoate, and (ii) a gold compound in the manufacture of a medicament for reducing inflammation.

In the fifth aspect, the inflammation may be associated with dermatitis or psoriasis. The dermatitis may be selected from the group consisting of atopic dermatitis, contact dermatitis, and seborrhoeic dermatitis.

In the present invention, the gold compound is preferably auranofin. Preferably the gold compounds used in the present invention are lipid soluble. Even more preferably the gold compounds used are formulated for topical application. However, it will be understood that systemically or locally administered compositions are also within the scope of the present invention including those administered by injection, preferably intra-articularly. In this regard, the corticosteroid can be formulated for oral, topical, systemic or local administration.

## BRIEF DESCRIPTION OF FIGURES

Figure 1: A histogram showing the effects of auranofin and glucocorticoids alone or in combination on TPA-induced epidermal hyperplasia. BMD: betamethasone dipropionate; HYD: hydrocortisone; FA: fluocinolone acetonide, MMF: mometasone furoate; AF: auranofin. Bars indicate standard error of the mean (SEM).

Figure 2: A histogram showing the effects of auranofin and glucocorticoids alone or in combination on TPA-induced inflammatory cell infiltration. BMD: betamethasone dipropionate; HYD: hydrocortisone; FA: fluocinolone acetonide; MMF: mometasone furoate; AF: auranofin. Bars indicate standard error of the mean (SEM).

## DESCRIPTION OF THE PREFERRED EMBODIMENT

For convenience, a TPA (12-*O*-tetradecanoylphorbol 13-acetate), model of psoriasis, as an example of an immune-mediated disorder which has an inflammatory as well as a cellular hyperproliferation component, will be used to demonstrate differential action of different corticosteroids as well as differential action of different formulations of a gold compound and a corticosteroid.

Although psoriasis does not occur in animals other than humans, studies have shown that the application of TPA produces an inflammatory reaction with epidermal thickening that resembles psoriasis in many ways. It produces epidermal hyperplasia and inflammatory cell infiltration into the dermis, both of these features are also characteristic of psoriasis.

TPA increases the activity of the phospholipase C/inositol trisphosphate/diacylglycerol system. This system activates the protein kinase C and arachidonic acid pathways. Both these systems have been implicated in the pathogenesis of psoriasis. TPA-treated mouse is believed to be a suitable model for psoriasis. This animal model will be used to show differential actions of different corticosteroids and the synergistic effects of compositions comprising a gold compound and a corticosteroid.

Three parameters have been measured: skin-fold thickness, epidermal hyperplasia and inflammatory cell infiltration into the dermis. These features, particularly the last two, are the hallmarks of psoriasis. It is noteworthy however, that the current findings clearly have implications beyond mere treatment of dermatological disorders. Systemic or tissue inflammatory and hyperplastic conditions could also benefit from treatment with the compositions of the present invention.

Topical Corticosteroids

Topical corticosteroids can be grouped according to their strength: weak, medium, strong and very strong. Vasoconstriction assay is considered the best method of assessing the potency of various preparations. It is not known whether the measurement of vasoconstriction predicts anti-inflammatory activity. Other methods of assaying that are available include clinical trial, dermal thickness radiograph, biopsy for assessing epidermal thinning, and mitotic inhibition assays.

A useful clinical guide to the relative potencies of topical corticosteroid preparations is shown in Table 1, the rank order arrangement being approximately the same for ointments and creams. The preparations in each group are only roughly equipotent.

**Table I** A guide to the clinical potencies of topical corticosteroids

| Weak | Medium | Strong | Very Strong |
|---|---|---|---|
| Dexamethasone 0.01% | Alclometasone dipropionate 0.05% | Amcinonide 0.1% | Beclomethasone dipropionate 0.5% |
| Fluocinolone acetonide 0.0025% | Betamethasone valerate 0.025% | Beclomethasone dipropionate 0.025% | Clobetasol propionate 0.05% |
| Hydrocortisone 0.5% and 0.1% | Clobetasone butyrate 0.05% | Betamethasone benzoate 0.025% | Diflucortolone valerate 0.3% |
| Hydrocortisone acetate 1% | Dexamethasone 0.05% | Betamethasone dipropionate 0.05% | Fluocinolone acetonide 0.2% |
| Methylprednisolone acetate 0.25% | Flumethasone pivalate 0.02% | Betamethasone valerate 0.1% | |
| | Fluocinolone acetonide 0.01% | Budesonide 0.025% | |
| | Fluocortin butylester 0.75% | Desonide 0.05% | |
| | Fluocortolone 0.2% | Dexamethasone 0.25% | |
| | Flurandrenolone 0.0125%-0.025% | Diflorasone diacetate 0.05% | |
| | Hydrocortisone 1% with urea | Diflucortolone valerate 0.1% | |
| | | Fluclorolone acetonide 0.025% | |
| | | Fluocinolone acetonide 0.025% | |
| | | Fluocinonide 0.05% | |
| | | Fluocortolone 0.5% | |
| | | Fluprednidene acetate 0.1% | |
| | | Flurandrenolone 0.05% | |
| | | Halcinonide 0.1% | |
| | | Hydrocortisone butyrate 0.1% | |
| | | Mometasone furoate 0.1% | |
| | | Triamcinolone acetonide 0.1% | |

The invention will now be more particularly described with reference to specific embodiments by way of non-limiting example only.

**Example 1: Animal treatment and methods of measurement**

Materials

Auranofin was kindly donated by Smith Kline and Beecham Pharmaceuticals, King of Prussia, Philadelphia, USA.
Alclometasone dipropionate, betamethasone dipopionate, betamethasone valerate, betamethasone valerate, betamethasone (as free alcohol) and mometasone furoate were kindly donated by Bristol-Myers Squibb Pharmaceuticals Pty.Ltd.
Dexamethasone was kindly donated by Roussel Uclaf, Paris, France.
Fluocortolone 21-pivalate was kindly donated by Schering AG, Berlin, Germany.
12-*O*-Tetradecanoylphorbol 13-acetate, fluocinolone acetonide, aluminium potassium sulphate, sodium hydrogen carbonate, formaldehyde acetic acid (17M), thymol, xylene, haematoxylin and "Paraplast" tissue embedding medium were obtained from Sigma Chemical Company, Castle Hill, NSW, Australia.
Sorbolene cream A.P.F. was obtained from Wille Laboratories, Carole Park, Queensland, Australia.

Preparation of auranofin 0.2% solution

Auranofin (20 mg) was dissolved in 10 mL of acetone to give the strength 0.2 %. Auranofin solution was freshly prepared for each experiment.

Preparation of auranofin ointment

Various strengths of auranofin ointment were made according to the formula as shown below:

| strength (% w/w) | auranofin | propylene glycol (10%) | white soft paraffin |
|---|---|---|---|
| 0.20 | 40 mg | 2 g | to 20 g |
| 0.50 | 100 mg | 2 g | to 20 g |

Methods of animal treatment

Female BALB/c mice aged 6 to 8 weeks were obtained from the University of Sydney, and treated according to a protocol approved by the University of Sydney Animal Care and Ethics Committee.
The mice were housed in stainless steel cages, 6 mice per cage under normal laboratory conditions (room temperature at about 22°C) at least 7 days before the experiments for acclimatization. Food and water were allowed *ad libitum* throughout the experiment period. The backs of the mice were shaved with an electric clipper two days before each treatment and only those mice showing no hair regrowth were used (i.e., the mice in the resting phase of the hair growth cycle were selected). During treatment, the mice were held with their tails and put on top of the cage so that they grasped the cage and rested there. The solutions were applied to an area approximately 2 cm x 2 cm on the shaved back of the mice by using a "Pipetman" to apply the solution. If auranofin ointment or ointment base was applied, the amount was standardized by using a microspatula which was crimped at one end, the ointment was then put into the ridge and the excess removed by means of another microspatula and applied sparingly twice a day. After a fixed time, the mice were killed routinely by cervical dislocation between 9 a.m. and I 1 a.m. to avoid variations due to circadian rhythms, and an area (1 cm x 1 cm) was excised from the centre of the treated area by scalpel and scissors. The rest of the tissues were disposed by combustion. The tissues were then fixed, embedded, sectioned and stained.

Methods of preparation of skin sections

The method of preparation of skin sections was adapted from a method developed by the Department of pathology, University of Sydney and shown to be successful.

Fixation, embedding, sectioning and staining

Standard preparation procedures were used. Briefly, the tissue was fixed in 10% buffered formalin for 24 hours, washed in tap water for 10 minutes and then processed in an automatic tissue processor (Tissue-Tek VIP 200). In the automatic tissue processor, the tissue was dehydrated in a graded series of alcohol and xylene at room temperature, and was then infiltrated with 4 changes of paraffin wax ("Paraplast" tissue mounting medium) at 60°C. It was finally embedded in fresh paraffin wax.

Sections 5 μm thick were cut on an American Optical Spencer "820" microtome. The sections were then mounted on clean microscope slides using wood glue ("Selleys" Aquadhere, 1:100 dilution with water) as adhesive, and were allowed to dry in an oven at 45°C for at least 2 hours (usually overnight).

After the sections were blued in the Scott's blueing solution, they were examined under microscope to assess that nuclei were clearly stained and cytoplasm was unstained.

Measurement of skin-fold thickness

The back skin of shaved mice was folded and measured by using a "Etalon" micrometer screw gauge. One measurement was taken for each mouse.

Measurement of epidermal thickness

Epidermal thickness was determined by image analysis. This image analysis system was a minicomputer (Tracor Northern TN8500) attached to a light microscope (Zeiss Axioplan) and a camcorder (Sony DXC-3000P). Sections were taken from each tissue block and 20 measurements were taken at fixed intervals from each section. The average value for the 20 measurements was obtained and entered as one value for each mouse. The mean and SEM for the six mice in each treatment group were calculated.

Measurement of infiltration of inflammatory cells

Infiltration of inflammatory cells was determined by the same image analysis system using the section taken from the block of mouse skin embedded in paraffin wax or Spurr's resin and stained with haematoxylin and eosin or toluidine blue respectively. For each section, 10 fields, unless otherwise stated, were chosen randomly and the cell density per $mm^2$ of field determined. The average value for the 10 fields was obtained and entered as one value for each mouse. The mean and SEM for the six mice in each treatment group were calculated.

The measurement of the inflammatory cell infiltration included the background values which included other materials in the dermis stained in the same way.- However, the increase, if there is any, reflects the migration of inflammatory cells.

Data treatment

The per cent inhibition of drug on TPA-induced skin responses (i.e., epidermal hyperplasia, inflammatory cell infiltration and skin-fold thickness) was calculated by using the following formula:

$$\text{Per cent inhibition} = \frac{\text{Total response due to TPA} - \text{Total response due to drug}}{\text{Total response due to TPA} - \text{Total response due to acetone}} \times 100\%$$

It is commonly found that the relationship between dose (or concentration) and response may be satisfactorily described using the Michaelis Menton equation or a variant of it such as the Hill equation. This provides estimates of (a) the potency, (b) the efficacy or maximum effect ($E_{max}$) and the slope of the log concentration-response curve by using Hill coefficient (γ). The Hill Equation may be expressed as follows:

$$E = \frac{Emax \times C^{\gamma}}{IC_{50}{}^{\gamma} + C^{\gamma}}$$

where $IC_{50}$ is the drug concentration producing 50% of the maximal response and $E_{max}$ refers to the maximal effect produced by the drug and is also termed efficacy. Efficacy is the measurement of the intrinsic ability of a drug to initiate a response once it occupies receptor sites. Measurement of both the $E_{max}$ and the $IC_{50}$ (potency) are clearly crucial when comparing the activity of similar drugs. The Hill coefficient ($\gamma$) measures the slope of the dose-effect curve which can be markedly influenced by the shape of the curve that describes the binding of the drug to the receptor. For many drugs, $\gamma$ lies between 0.6 and 1.5. The use of the Hill coefficient not only improves the fit of the data, but also indicates the influence of changes in dose or concentration on response: for example, when $\gamma$ is greater than 1, the slope is very steep, meaning that a marked change in drug effect is associated with a small change in dose or concentration of drug. On the other hand, when $\gamma$ is less than 1, with a shallow hyperbolic concentration effect relationship, the activity occurs over a wide range of drug levels. Hence, the different values of $\gamma$ can dramatically affect the drug's clinical usefulness

In the present study, concentration-response curves were obtained for a series of corticosteroids. In these experiments, curve fitting was accomplished using a computer programme called "The Scientist" (MicroMath Scientific Software, Salt Lake City, Utah, USA.) in which the parameters, $E_{max}$, $IC_{50}$ and Hill coefficient ($\gamma$) for each steroids were estimated by using non-linear regression and least square fits.

The overall significance of differences between treatments was determined by one way analysis of variance, while the Tukey HSD test was used to examine the significance level of specific contrasts. The Systat for Windows program (Systat Inc, Evanston, Illinois, USA.) was used.

**Example 2: Effects of TPA on skin of mice**

This study was conducted to determine the time course of effects produced by applying TPA to mice and killing at intervals of 1. 2. 3. 5 and 8 days. Peak times for epidermal hyperplasia, dermal inflammation and skin-fold thickness were determined. The object of this study was to determine the best time to sacrifice TPA-treated mice so as to obtain the maximum response to TPA. The literature indicates that TPA-induced epidermal hyperplasia and dermal inflammation peak at different times. It was thus expected that a compromise time would have to be selected.

Forty two female BALB/c strain mice were divided into the treatment groups and the mice were treated with a single application (100 μL) of TPA (0.01% in acetone) and sacrificed at days 1, 2, 3, 5 and 8 and the time course of TPA effects on epidermal hyperplasia, dermal inflammatory cell infiltration and skin-fold thickness measured as described in Example 1.

The skin responses to TPA are summarized below:

| | Day 1 | Day 2 | Day 3 | Day 5 | Day 8 |
|---|---|---|---|---|---|
| Increase in epidermal thickness | 326% | 382% | 393% | 270% | 130% |
| Increase in dermal inflammatory cell density | 397% | 296% | 272% | 280% | 225% |
| Increase in skin-fold thickness | 193% | 142% | 124% | 116% | 107% |

The experiment showed that a single application of TPA caused epidermal thickening, dermal inflammation and an increase in skin-fold thickness that lasted for at least 8 days. The peak effects were observed at 72 hours for epidermal hyperplasia and at 24 hours for dermal inflammation and skin-fold thickening.

Based on these results it was decided that an appropriate compromise, for most experiments, would be to sacrifice the animals 24 hours after a single application of TPA. This would result in maximum effects for inflammation and skin-fold thickness and near maximum effects for epidermal hyperplasia (over 80% of peak effect). Unless otherwise stated, mice were sacrificed 24 hours after the application of TPA.

**Example 3: Action of Different Corticosteroids**

In the first instance the ability of corticosteroids alone to inhibit TPA lesions was investigated. The following groups of corticosteroids were tested (the classification of the potencies of corticosteroids is dependent on the concentration used, the composition of the vehicle and the effect being studied) (Table 1).

**Low potency:** betamethasone, dexamethasone, hydrocortisone, hydrocortisone acetate.

**Medium potency:** alclometasone dipropionate, fluocortolone 21-pivalate.

**High potency:** betamethasone dipropionate, betamethasone valerate, fluocinolone acetonide, halcinonide, mometasone furoate, triamcinolone acetonide

Mice were divided into treatment groups. TPA and corticosteroids were premixed to the concentrations required and applied to the backs of mice immediately. For those steroids not very soluble in acetone (i.e., betamethasone, dexamethasone, hydrocortisone and hydrocortisone acetate), the drugs were dissolved in 100 μL dimethylformamide before further dilution with acetone. The efficacy of corticosteroids in inhibiting epidermal hyperplasia, inflammatory cell infiltration and skin-fold thickness was assessed as described previously. Concentration-response curves were determined after computer-fitting of data. The curve fitting technique was generated by a computer programme called "The Scientist" in which the parameters in the Hill Equation were generated. These included $E_{max}$. $IC_{50}$, and the Hill coefficient ($\gamma$) for each steroid. Concentration-response curves for each steroid were plotted and the relative potencies were determined from the $IC_{50}$ values. Comparisons were made of variations in ratios of $IC_{50}$ for each corticosteroids tested. These values were compared with those in the literature and reflected the relative intrinsic potencies of steroids.

**Table 2 A summary of the concentration-response curves for inhibition of TPA-induced epidermal hyperplasia by various steroids showing the values of $E_{max}$, $IC_{50}$ and gamma ($\gamma$) with respect to the Hill equation.**

| Corticosteroids | $E_{max}$ (% inhibition) | $IC_{50}$ (M x $10^{-4}$) | Gamma ($\gamma$) |
|---|---|---|---|
| Alclometasone dipropionate | 96.59 ± 7.90 (76.28-116.90) | 0.89 ± 0.30 (0.16-1.67) | 0.67 ± 0.12 (0.36-0.97) |
| Betamethasone | 94.19 ± 7.33 (75.35-113.04) | 13.81 ± 3.73 (4.22-23.39) | 0.81 ± 0.11 (0.51-1.10) |
| Betamethasone dipropionate | 95.59 ± 6.55 (82.14-109.04) | 0.81 ± 0.36 (0.49-1.13) | 0.54 ± 0.06 (0.37-0.71) |
| Betamethasone valerate | 82.24 ± 2.84 (75.69-88.78) | 0.15 ± 0.02 (0.10-0.20) | 0.94 ± 0.12 (0.66-1.21) |
| Dexamethasone | 90.70 ± 5.72 (76.72-104.69) | 1.26 ± 0.44 (0.85-1.67) | 0.50 ± 0.08 (0.31-0.69) |
| Fluocinolone acetonide | 89.60 ± 6.93 (70.36 ± 108.85) | 0.39 ± 0.14 (0.043-0.78) | 0.75 ± 0.23 (0.12-1.38) |
| Fluocortolone 21-pivalate | 85.18 ± 9.51 (58.79-111.58) | 0.40 ± 0.15 (0.12-0.68) | 0.97 ± 0.30 (0.13-1.82) |
| Halcinonide | 92.55 ± 2.98 (85.25-99.85) | 0.97 ± 0.13 (0.66-1.27) | 0.78 ± 0.06 (0.62-0.94) |
| Hydrocortisone | 99.04 ± 9.54 (72.56-125.51) | 46.18 ± 13.22 (9.47-82.90) | 0.96 ± 0.17 (0.49-1.44) |
| Hydrocortisone acetate | 79.63 ± 7.62 (60.04-99.23) | 36.67 ± 12.01 (5.80-67.56) | 0.79 ± 0.13 (0.46-1.13) |
| Mometasone furoate | 83.31 ± 3.04 (76.11-90.51) | 0.23 ± 0.04 (0.14-0.33) | 0.79 ± 0.11 (0.53-1.04) |
| Triamcinolone acetonide | 89.80 ± 6.40 (74.14-105.46) | 1.13 ± 0.35 (0.28-1.98) | 0.75 ± 0.15 (0.39-1.11) |
| Results are presented as means ± SD. Figures in brackets are confidence intervals | | | |

**Table 3 A summary of the concentration-response curves for inhibition of TPA-induced inflammatory cell infiltration by various steroids showing the values of $E_{max}$, $IC_{50}$ and gamma ($\gamma$) with respect to the Hill equation.**

| Corticosteroids | $E_{max}$ (% inhibition) | $IC_{50}$ (M x $10^{-4}$) | Gamma ($\gamma$) |
|---|---|---|---|
| Alclometasone dipropionate | 82.12 ± 5.65 (67.58-96.66) | 0.32 ± 0.09 (0.10-0.54) | 0.97 ± 0.26 (0.31-1.63) |
| Betamethasone | 74.55 ± 3.29 (66.09-83.01) | 13.12 ± 1.65 (8.87 ± 17.37) | 1.28 ± 0.17 (0.85-1.71) |
| Betamethasone dipropionate | 90.77 ± 3.71 (83.18-98.37) | 0.29 ± 0.06 (0.16-0.41) | 0.52 ± 0.06 (0.39-0.65) |
| Betamethasone valerate | 73.52 ± 1.76 (69.46-77.57) | 0.29 ± 0.03 (0.22-0.34)) | 0.93 ± 0.07 (0.77-1.09) |
| Dexamethasone | 99.05 ± 10.98 (72.18-125.93) | 4.44 ± 1.02 (3.19-5.69) | 0.73 ± 0.19 (0.25-1.20) |
| Fluocinolone acetonide | 79.60 ± 2.41 (74.05-85.15) | 0.16 ± 0.02 (0.11-0.21) | 1.05 ± 0.14 (0.72-1.38) |
| Fluocortolone 21-pivalate | 97.91 ± 12.16 (64.15 ± 131.67) | 1.10 ± 0.40 (0.55-1.65) | 0.94 ± 0.21 (0.36-1.54) |
| Halcinonide | 90.34 ± 6.29 (74.94-105.74) | 0.31 ± 0.05 (0.14-0-48) | 0.60 ± 0.11 (0.32-0.88) |
| Hydrocortisone | 100.29 ± 9.36 (71.35-129.23) | 61.48 ± 9.66 (39.13-83.83) | 0.57 ± 0.26 (0.25-0.89) |
| Hydrocortisone acetate | 79.36 ± 11.49 (59.39-99.33) | 77.33 ± 13.85 (60.28-94.58) | 1.11 ± 0.27 (0.42-1.80) |
| Mometasone furoate | 89.98 ± 4.88 (78.45-101.51) | 0.20 ± 0.05 (0.08-0.33) | 0.69 ± 0.14 (0.37-1.01) |
| Triamcinolone acetonide | 77.24 ± 1.26 (74.15-80.33) | 0.39 ± 0.03 (0.33-0.46) | 1.42 ± 0.11 (1.14-1.70) |
| Results are presented as means ± SD. Figures in brackets are confidence intervals | | | |

Tables 2 and 3 provide an estimate of the efficacies ($E_{max}$), potencies ($IC_{50}$) and slope of the concentration-response curve ($\gamma$) for the 12 corticosteroids investigated in this study. These values were obtained from the Hill equation which is a modified form of the Michaelis-Menton equation.

The apparent excellent correlations between $IC_{50}$ values could indicate that inhibition of inflammation and epidermal hyperplasia are mediated by the same mechanism or else that the limiting factor in producing the two effects was the ability of the steroid to penetrate the skin. The fact that the maximum effects ($E_{max}$) and slopes ($\gamma$) of the concentration-response curves for the two effects were very poorly correlated suggests that different mechanisms are involved in suppressing inflammation and epidermal hyperplasia by the steroids. In addition, the correlations between $IC_{50}$ values are not so impressive when the effects of certain outlier drugs are removed. The effect of removing outliers is shown in Table 4:

**Table 4: $IC_{50}$ values (inflammation vs epidermal hyperplasia)**

| **Plot of $IC_{50}$ values (inflammation vs epidermal hyperplasia)** | **r** (derived from Hill equation) | **r** (read from graph where $IC_{50}$ = concentration that inhibits half the effect of TPA) |
|---|---|---|
| All drugs included | 0.961 (p<0.0005) | 0.909 (p<0.0005) |
| Hydrocortisone and hydrocortisone acetate not included | 0.959 (p<0.0005) | 0.988 (p<0.0005) |
| Hydrocortisone, hydrocortisone acetate, betamethasone dipropionate, dexamethasone and fluocortolone 21-pivalate not included | 0.74 1 (p = 0.057) | 0.637 (p = 0.072) |

From the above results it seems likely that the steroids inhibit the inflammatory and hyperplastic effects of TPA by different mechanisms, either inducing different biochemical responses or producing the same response in different cell lines. This conclusion is very relevant to the possible synergistic effects of auranofin are described.

The determination of $E_{max}$ values from the Hill equation could be subject to error due to some uncertainty about measurements made at the top of the concentration-response curves. Therefore, $IC_{50}$ values were determined by two methods: (a) using the $E_{max}$ value generated from the Hill equation (Tables 5 and 6), and (b) directly from the concentration-response curve, taking the $IC_{50}$ value as that concentration that inhibited 50% of the TPA-induced hyperplasia and inflammation (Tables 7 and 8). The difference between $IC_{50}$ values, and hence relative potencies, determined by the two methods was not great. However, direct reading from the concentration-response curve was considered more reliable and these readings were used for calculating the "synergistic factors" given in Tables 9 and 10.

**Table 5 Actual and relative potencies of topical corticostcroids for inhibition of TPA-induced epidermal hyperplasia [values generated from the Hill equation where $IC_{50}$ is the concentration that produced 50% of the maximum inhibitory effect ($E_{max}$)].**

| **Corticosteroids** | **$IC_{50}$ for inhibition of epidermal thickening ($M \times 10^{-4}$)** | **Relative potency** |
|---|---|---|
| **Hydrocortisone acetate** | 36.67 | 1.0 |
| **Hydrocortisone** | 46.18 | 0.79 |
| **Betamethasone** | 27.25 | 1.35 |
| **Dexamethasone** | 1.26 | 29.10 |
| **Triamcinolone acetonide** | 1.13 | 32.45 |
| **Halcinonide** | 0.97 | 37.80 |
| **Alclometasone dipropionate** | 0.89 | 41.20 |
| **Betamethasone dipropionate** | 0.81 | 45.27 |
| **Fluocortolone 21-pivalate** | 0.4 | 91.68 |
| **Fluocinolone acetonide** | 0.39 | 94.03 |
| **Mometasone furoate** | 0.23 | 159.43 |
| **Betamethasone valerate** | 0.15 | 244.47 |

**Table 6 Actual and relative potencies of topical corticosteroids for inhibition of TPA-induced inflammatory cell infiltration into the dermis [values generated from the Hill equation where $IC_{50}$ is the concentration that produced 50% of the maximum inhibitory effect ($E_{max}$)].**

| Corticosteroids | $IC_{50}$ for inhibition of inflammatory cell infiltration in the dermis (M x $10^{-4}$) | Relative potency |
|---|---|---|
| Hydrocortisone acetate | 77.33 | 1.0 |
| Hydrocortisone | 61.48 | 1.26 |
| Betamethasone | 13.12 | 5.89 |
| Dexamethasone | 4.44 | 17.42 |
| Fluocortolone 21-pivalate | 1.10 | 70.30 |
| Triamcinolone acetonide | 0.39 | 198.28 |
| Alclometasone dipropionate | 0.32 | 241.66 |
| Halcinonide | 0.31 | 249.45 |
| Betamethasone dipropionate | 0.29 | 266.66 |
| Betamethasone valerate | 0.29 | 266.66 |
| Mometasone furoate | 0.20 | 386.65 |
| Fluocinolone acetonide | 0.16 | 483.31 |

**Table 7 Actual and relative potencies of topical corticosteroids for inhibition of TPA-induced epidermal hyperplasia (values taken from concentration-response curve where $IC_{50}$ is the concentration that inhibited 50% of the TPA effect).**

| Corticosteroids | $IC_{50}$ for inhibition of epidermal thickening (M x $10^{-4}$) | Relative potency |
|---|---|---|
| Hydrocortisone acetate | 70.00 | 1.0 |
| Hydrocortisone | 45.10 | 1.55 |
| Betamethasone | 50.00 | 1.40 |
| Dexamethasone | 2.00 | 35.0 |
| Triamcinolone acetonide | 1.40 | 50.0 |
| Betamethasone dipropionate | 1.40 | 50.0 |
| Halcinonide | 1.10 | 63.64 |
| Alclometasone dipropionate | 1.00 | 70.0 |
| Fluocortolone 21-pivalate | 0.60 | 116.67 |
| Mometasone furoate | 0.40 | 175.0 |
| Betamethasone valerate | 0.22 | 318.18 |
| Fluocinolone acetonide | 0.21 | 333.33 |

**Table 8 Actual and relative potencies of topical corticosteroids for inhibition of TPA-induced inflammatory cell infiltration into the dermis (values taken from concentration-response curve where $IC_{50}$ is the concentration that inhibited 50% of the TPA effect).**

| Corticosteroids | $IC_{50}$ for inhibition of inflammatory cell infiltration in the dermis (M $\times$ $10^{-4}$) | Relative potency |
|---|---|---|
| Hydrocortisone acetate | 110.0 | 1.0 |
| Hydrocortisone | 60.0 | 1.83 |
| Betamethasone | 21.0 | 5.23 |
| Dexamethasone | 4.10 | 26.83 |
| Fluocortolone 21-pivalate | 1.10 | 100.0 |
| Triamcinolone acetonide | 0.70 | 157.14 |
| Betamethasone valerate | 0.65 | 169.23 |

Table continued

| Corticosteroids | $IC_{50}$ for inhibition of inflammatory cell infiltration in the dermis ($M \times 10^{-4}$) | Relative potency |
|---|---|---|
| Alclometasone dipropionate | 0.60 | 183.33 |
| Halcinonide | 0.60 | 183.33 |
| Betamethasone dipropionate | 0.40 | 275.0 |
| Mometasone furoate | 0.30 | 366.67 |
| Fluocinolone acetonide | 0.25 | 440.0 |

In the present study, hydrocortisone, hydrocortisone acetate, betamethasone and dexamethasone had low to medium potencies with respect to both inhibition of TPA-induced epidermal hyperplasia and TPA-induced inflammatory cell infiltration. This ranking was consistent with corresponding potencies in clinical setting that was shown in Table 1.

Halcinonide, triamcinolone acetonide, alclometasone dipropionate, betamethasone dipropionate, and fluocortolone 21-pivalate were found to have medium to strong potencies in inhibition of TPA-induced epidermal hyperplasia and inflammatory cell infiltration, and these values were consistent with the clinical potencies.

Finally, mometasone furoate, betamethasone valerate, betamethasone dipropionate, and fluocinolone acetonide had strong to very strong potencies for TPA-induced epidermal hyperplasia. Amongst them, betamethasone valerate was shown to be the most potent agent. When their potencies for inhibition of TPA-induced inflammatory cell infiltration were investigated, they were also shown to have strong to very strong anti-inflammatory effect. Fluocinolone acetonide was found to be the most potent agent, with a relative potency of 483 (Table 6) or 440 (Table 8).

When comparisons were made between the relative potencies of the steroids tested in this study and their respective clinical potencies, the order of potency was generally the same.

It might be expected that a particular corticosteroid would have equal potency with respect to anti-hyperplastic and anti-inflammatory actions. Our results showed that these reactions were not necessarily closely related. Linear regression of the relative potencies for inhibition of the hyperplastic effects of the steroids vs relative potencies for suppression of inflammation gave a value $r = 0.573$ ($p = 0.51$) for data from tables 5 and 6 and a value of $r = 0.658$ ($p<0.02$) for values in tables 7 and 8. Thus, about 65% of the variance between the two actions seems to be due to some common property (which could be lipid solubility) but a significant component of these actions differs with respect to suppression of hyperplasia and suppression of inflammation. This is also illustrated by comparing the rank orders of potency in Tables 7 and 8. Only six of the 12 steroids have the same rank for both effects and, of these steroids, four are the four least potent.

**Example 4: Synergistic Effect Of Auranofin And Corticosteroids With Different Clinical Potencies**

Results of preliminary studies indicated that auranofin, under certain conditions, can inhibit some of the effects of TPA, although it is not particularly potent in this regard.

The present study examines whether combinations of auranofin and corticosteroids had a synergistic effect in suppressing TPA lesions.

Four corticosteroids with different clinical potencies ranging from weak to strong were chosen, namely, hydrocortisone, fluocinolone acetonide, betamethasone dipropionate and mometasone furoate. TPA was premixed with the corticosteroids in the presence or absence of a fixed concentration ofauranofin (0.2%) to produce the required concentrations and applied to the backs of mice immediately. Concentration-response curves were determined by non-linear regression using least squares fitting. $IC_{50}$ values were determined by reading the value from the graph that corresponded to 50% of the effect produced by TPA (Tables 9 and 10).

The term 'apparent $IC_{50}$' refers to the value for the combination of steroid and auranofin.

**Table 9 Apparent $IC_{50}$ values obtained from the computer fitted graph for the effects on TPA-induced epidermal hyperplasia of four corticosteroids in the absence and presence of auranofin (0.2%).**

| | Apparent $IC_{50}$ ($M \times 10^{-4}$) | | |
|---|---|---|---|
| Corticosteroids | without auranofin | with auranofin | Synergistic factor |
| Betamethasone dipropionate | $1.40 \pm 0.36$ | $0.025 \pm 0.003$ | 56.0 |
| Hydrocortisone | $45.00 \pm 13.22$ | $22.00 \pm 11.23$ | 2.05 |
| Fluocinolone acetonide | $0.21 \pm 0.14$ | $0.016 \pm 0.002$ | 13.13 |

Table continued

| | Apparent $IC_{50}$ (M x $10^{-4}$) | | |
|---|---|---|---|
| **Corticosteroids** | **without auranofin** | **with auranofin** | **Synergistic factor** |
| **Mometasone furoate** | 0.40 ± 0.04 | 0.31 ± 0.05 | 1.29 |
| Results are presented as means ± SD. | | | |

**Table 10 Apparent $IC_{50}$ values obtained from the computer fitted graph for the effects on TPA-induced dermal inflammatory cell infiltration of four corticosteroids in the absence and presence of auranofin (0.2%).**

| | Apparent $IC_{50}$ (M x $10^{-4}$) | | |
|---|---|---|---|
| **Corticosteroids** | **without auranofin** | **with auranofin** | **Synergistic factor** |
| **Betamethasone dipropionate** | 0.40 ± 0.06 | 0.090 ± 0.016 | 4.44 |
| **Hydrocortisone** | 60.00 ± 9.66 | 31.00 ± 4.12 | 1.94 |
| **Fluocinolone acetonide** | 0.25 ± 0.002 | 0.11 ± 0.013 | 2.27 |
| **Mometasone furoate** | 0.30 ± 0.05 | 0.008 ± 0.001 | 37.50 |
| Results are presented as means ± SD. | | | |

The value termed the 'synergistic factor' is defined as the $IC_{50}$ value for the steroid determined in the absence of auranofin divided by the $IC_{50}$ for the same steroid determined in the presence of auranofin (0.2%).

Synergism refers to situations in which a combination of two drugs produces an effect that is significantly greater than the algebraic sum of the effects when the same dose or concentration of each drug is observed separately in the same test system. Synergism can result in a multifold potentiation of the effects of one or both drugs or it can give rise to effects that are qualitatively different from those elicited by the drugs when used separately.

With respect to effects on epidermal hyperplasia, only two of the four steroids tested could be regarded as showing a synergistic reaction with auranofin, namely betamethasone dipropionate and fluocinolone acetonide. From results depicted in Tables 9 and 10 it can be seen that in the case of betamethasone dipropionate and fluocinolone acetonide, the per cent increase in the apparent potencies of the steroids is 5.600% and 1,300%, respectively. However, in the case of the least potent of the four steroids, namely, hydrocortisone, the apparent increase in potency was 100%. A more effective way of demonstrating the presence of true synergism is to compare separately the following:

1) auranofin (0.2%) alone,
2) a low concentration of steroid alone (sufficient to inhibit about 20% of the effects of TPA - this value can be read from the concentration-response curve for the steroid when studied alone),
3) the same concentration of steroid as in (2) combined with auranofin (0.2%) — this value can be obtained from the dose-response curve for steroid in the presence of auranofin, and
4) a concentration of the same steroid used in (2) but in sufficient concentration as to produce the same effect as that achieved in (3).

If the effects of (3) appear to be the summation of (1) and (2), the result is not synergism. If the effects of (3) greatly exceed those of (1) and (2) if added arithmetically, the result is synergism according to our definition.
The results for epidermal hyperplasia are displayed in Figure 1. Auranofin alone inhibited TPA-induced epidermal hyperplasia by about 10% in all four studies. Betamethasone dipropionate $1 \times 10^{-5}$ M alone caused about 20% inhibition of TPA effect. The combination of betamethasone dipropionate ($1 \times 10^{-5}$ M) and auranofin (0.2%) produced about 65% inhibition of TPA effect. To gauge the significance of the increased effect that resulted when auranofin was added to betamethasone dipropionate, comparison should be made with the concentration of betamethasone dipropionate that, in the absence of auranofin, produced the same effect. This value was 5 x $10^{-4}$ M or 50 times the concentration that produced the same effect in the presence of auranofin.

With respect to epidermal hyperplasia, the studies demonstrate that: (a) a massive synergism results when auranofin is added to betamethasone dipropionate and to fluocinolone acetonide; (b) a minor degree of synergism may result -from the combination of hydrocortisone and auranofin; and (c) that no synergism or even additive effect results when auranofin is co-administered with mometasone furoate.

With respect to inflammatory cell infiltration into the dermis, the results in Table 10 indicate that the effects of a combination of auranofin (0.2%) and mometasone furoate is the result of synergism since the apparent $IC_{50}$ is increased

by about 3,800%. It is also possible that a lesser degree of synergism occurs with respect to the anti-inflammatory action of betamethasone dipropionate in the presence of 0.2% auranofin. Here the increase in apparent $IC_{50}$ is of the order of 400-500% (Table 10).

The results in Figure 2 show a massive synergism between mometasone furoate ($5 \times -10^{-6}$ M) and auranofin (0.2%). This combination produced an effect that was equal to that produced by I x $10^{-3}$ M mometasone in the absence of auranofin. Figure 2 also indicates that synergism may exist for the combination of auranofin with betamethasone dipropionate and fluocinolone acetonide, but not with hydrocortisone.

The results of these studies indicate that extensive synergism results from the combination of auranofin with certain corticosteroids, such as for example betamethasone dipropionate and fluocinolone acetonide as regards reduction of epidermal hyperplasia, and with others such as for example mometasone furoate as regards reduction of inflammation. Lesser degrees of synergism may exist between auranofin and other steroids.

Gold compounds and corticosteroids, as well as their formulations, which can be suitably used in the present invention have been discussed in detail in the present application or in Australian patent No. 616 755, which is incorporated herein by reference.

## Claims

1. A pharmaceutical composition comprising a gold compound and a corticosteroid selected from fluocinolone acetonide and mometasone furoate in combination with a pharmaceutically acceptable carrier, excipient, adjuvant or solvent.

2. A composition according to claim 1, wherein the composition is formulated for systemic administration, oral administration, local administration, or topical administration or for administration by intra-articular injection.

3. A composition according to claim 1 or claim 2, wherein the gold compound is auranofin.

4. Products containing a corticosteroid selected from fluocinolone acetonide and hydrocortisone, and a gold compound as a combined preparation for simultaneous, separate or sequential use in reducing epidermal hyperplasia.

5. Products according to claim 4, wherein the epidermal hyperplasia is associated with psoriasis.

6. Products containing a corticosteroid selected from fluocinolone acetonide and mometasone furoate, and a gold compound as a combined preparation for simultaneous, separate or sequential use in reducing inflammation.

7. Products as claimed in claim 6, wherein inflammation is reduced in a skin condition selected from the group consisting of atopic dermatitis, contact dermatitis, seborrhoeic dermatitis and psoriasis.

8. Products according to any one of claims 4 to 7, wherein the gold compound is auranofin.

9. The use of (i) a corticosteroid selected from fluocinolone acetonide and hydrocortisone, and (ii) a gold compound in the manufacture of a medicament for reducing epidermal hyperplasia.

10. The use as claimed in claim 9, wherein the epidermal hyperplasia is associated with psoriasis.

11. The use of (i) a corticosteroid selected from fluocinolone acetonide and mometasone furoate, and (ii) a gold compound in the manufacture of a medicament for reducing inflammation.

12. The use as claimed in claim 11, wherein inflammation is associated with dermatitis or psoriasis.

13. The use as claimed in claim 12, wherein dermatitis is selected from the group consisting of atopic dermatitis, contact dermatitis, seborrhoeic dermatitis

14. The use as claimed in any one of claims 8 to 13, wherein the gold compound is auranofin.

## Patentansprüche

1. Eine pharmazeutische Zusammensetzung, die eine Goldverbindung und ein Corticosteroid, ausgewählt aus Fluocinolonacetonid oder Mometasonfuorat, in Kombination mit einer pharmazeutisch geeigneten Trägersubstanz, einem Bindemittel, Zusatzmittel oder Lösungsmittel enthält.

2. Eine Zusammensetzung gemäß Anspruch 1, wobei die Zusammensetzung für eine systemische Verabreichung, orale Verabreichung, lokale Anwendung oder topische Anwendung oder für eine Verabreichung durch intraartikuläre Injektion formuliert wird.

3. Eine Zusammensetzung gemäß Anspruch 1 oder Anspruch 2, wobei es sich bei der Goldverbindung um Auranofin handelt.

4. Produkte, die ein Corticosteroid, ausgewählt aus Fluocinolonacetonid oder Hydrocortison, und eine Goldverbindung, als Kombinationspräparat für eine gleichzeitige, getrennte oder aufeinanderfolgende Anwendung zur Verminderung von epidermaler Hyperplasie enthalten.

5. Produkte gemäß Anspruch 4, wobei die epidermale Hyperplasie mit einer Psoriasis in Verbindung steht.

6. Produkte, die ein Corticosteroid, ausgewählt aus Fluocinolonacetonid oder Mometasonfuroat, und eine Goldverbindung als Kombinationspräparat für eine gleichzeitige, getrennte oder aufeinanderfolgende Anwendung zur Verminderung von Entzündungen enthalten.

7. Produkte wie in Anspruch 6 beansprucht, wobei die Entzündung auf einen Hauterkrankung begrenzt wird, die aus Neurodermitis, Kontaktdermatitis, seborrhoische Dermatitis und Psoriasis ausgewählt ist.

8. Produkte gemäß einem der Ansprüche 4 bis 7, wobei es sich bei der Goldverbindung um Auranofin handelt.

9. Verwendung eines (i) Corticosteroids, ausgewählt aus Fluocinolonacetonid oder Hydrocortison, und (ii) einer Goldverbindung zur Herstellung eines Medikaments zum Vermindern einer epidermalen Hyperplasie.

10. Verwendung nach Anspruch 9, wobei die epidermale Hyperplasie mit Psoriasis in Verbindung steht.

11. Verwendung eines (i) Corticosteroids, ausgewählt aus Fluocinolonacetonid oder Mometasonfuorat, und (ii) einer Goldverbindung zur Herstellung eines Medikaments zur Verminderung von Entzündungen.

12. Verwendung nach Anspruch 11, wobei die Entzündung mit einer Dermatitis oder Psoriasis in Verbindung steht.

13. Verwendung nach Anspruch 12, wobei es sich bei der Dermatitis um eine Erkrankung aus der Gruppe Neurodermitis, Kontaktdermatitis, seborrhoische Dermatitis handelt.

14. Verwendung nach einem der Ansprüche 8 bis 13, wobei es sich bei der Goldverbindung um Auranofin handelt.

## Revendications

1. Composition pharmaceutique comprenant un composé de l'or et un corticostéroïde choisi parmi l'acétonide de fluocinolone et le furoate de mométasone en combinaison avec un véhicule, excipient, adjuvant ou solvant pharmaceutiquement acceptable.

2. Composition selon la revendication 1, **caractérisée en ce que** la composition est formulée pour une administration systémique, une administration orale, une administration locale ou une administration topique ou pour une administration par injection intra-articulaire.

3. Composition selon la revendication 1 ou la revendication 2, **caractérisée en ce que** le composé de l'or est l'auranofine.

4. Produits contenant un corticostéroïde choisi parmi l'acétonide de fluocinolone et l'hydrocortisone, et un composé de l'or sous forme d'une préparation combinée pour une utilisation simultanée, séparée ou séquentielle dans la réduction d'une hyperplasie épidermique.

5. Produits selon la revendication 4, **caractérisés en ce que** l'hyperplasie épidermique est associée au psoriasis.

6. Produits contenant un corticostéroïde choisi parmi l'acétonide de fluocinolone et le furoate de mométasone, et un composé de l'or sous forme d'une préparation combinée pour une utilisation simultanée, séparée ou séquentielle dans la réduction d'une inflammation.

7. Produits selon la revendication 6, **caractérisés en ce que** l'inflammation est réduite en une affection cutanée choisie parmi le groupe constitué de la dermatite atopique, de l'eczéma de contact, de la dermatite séborrhéique et du psoriasis.

8. Produits selon l'une quelconque des revendications 4 à 7, **caractérisés en ce que** le composé de l'or est l'auranofine.

9. Utilisation (i) d'un corticostéroïde choisi parmi l'acétonide de fluocinolone et l'hydrocortisone et (ii) d'un composé de l'or dans la préparation d'un médicament pour réduire une hyperplasie épidermique.

10. Utilisation selon la revendication 9, **caractérisée en ce que** l'hyperplasie épidermique est associée au psoriasis.

11. Utilisation (i) d'un corticostéroïde choisi parmi l'acétonide de fluocinolone et le furoate de mométasone et (ii) d'un composé de l'or dans la préparation d'un médicament pour réduire une inflammation.

12. Utilisation selon la revendication 11, **caractérisée en ce que** l'inflammation est associée à une dermatite ou au psoriasis.

13. Utilisation selon la revendication 12, **caractérisée en ce que** la dermatite est choisie parmi le groupe constitué de la dermatite atopique, l'eczéma de contact et la dermatite séborrhéique.

14. Utilisation selon l'une quelconque des revendications 8 à 13, **caractérisée en ce que** le composé de l'or est l'auranofine.

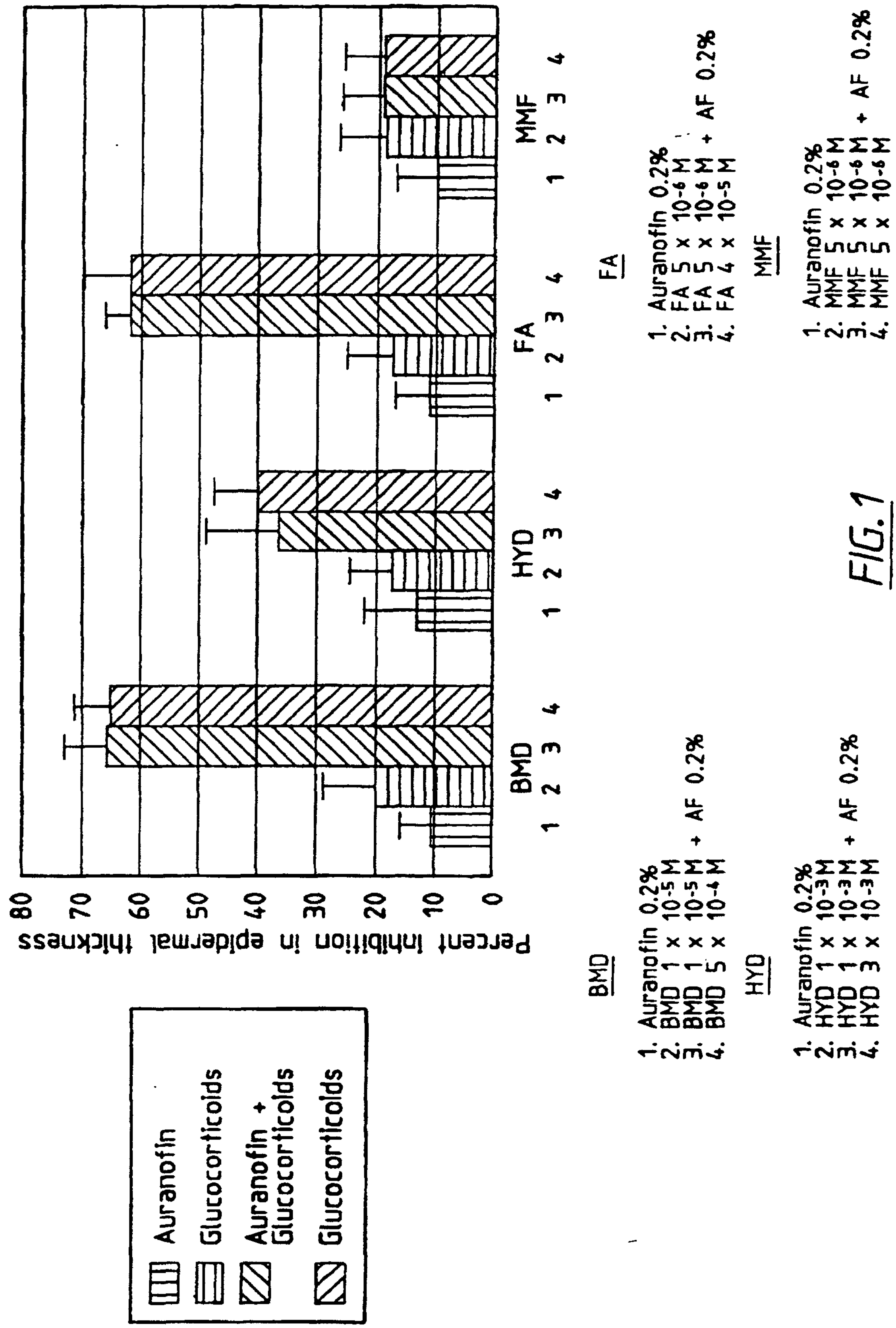
Auranofin
Glucocorticoids
Auranofin + Glucocorticoids
Glucocorticoids
Percent inhibition in epidermal thickness
0
10
20
30
40
50
60
70
80
BMD
1 2 3 4
HYD
1 2 3 4
FA
1 2 3 4
MMF
1 2 3 4
BMD
1. Auranofin 0.2%
2. BMD 1 x 10-5 M
3. BMD 1 x 10-5 M + AF 0.2%
4. BMD 5 x 10-4 M
HYD
1. Auranofin 0.2%
2. HYD 1 x 10-3 M
3. HYD 1 x 10-3 M + AF 0.2%
4. HYD 3 x 10-3 M
FA
1. Auranofin 0.2%
2. FA 5 x 10-6 M
3. FA 5 x 10-6 M + AF 0.2%
4. FA 4 x 10-5 M
MMF
1. Auranofin 0.2%
2. MMF 5 x 10-6 M
3. MMF 5 x 10-6 M + AF 0.2%
4. MMF 5 x 10-6 M

FIG. 1

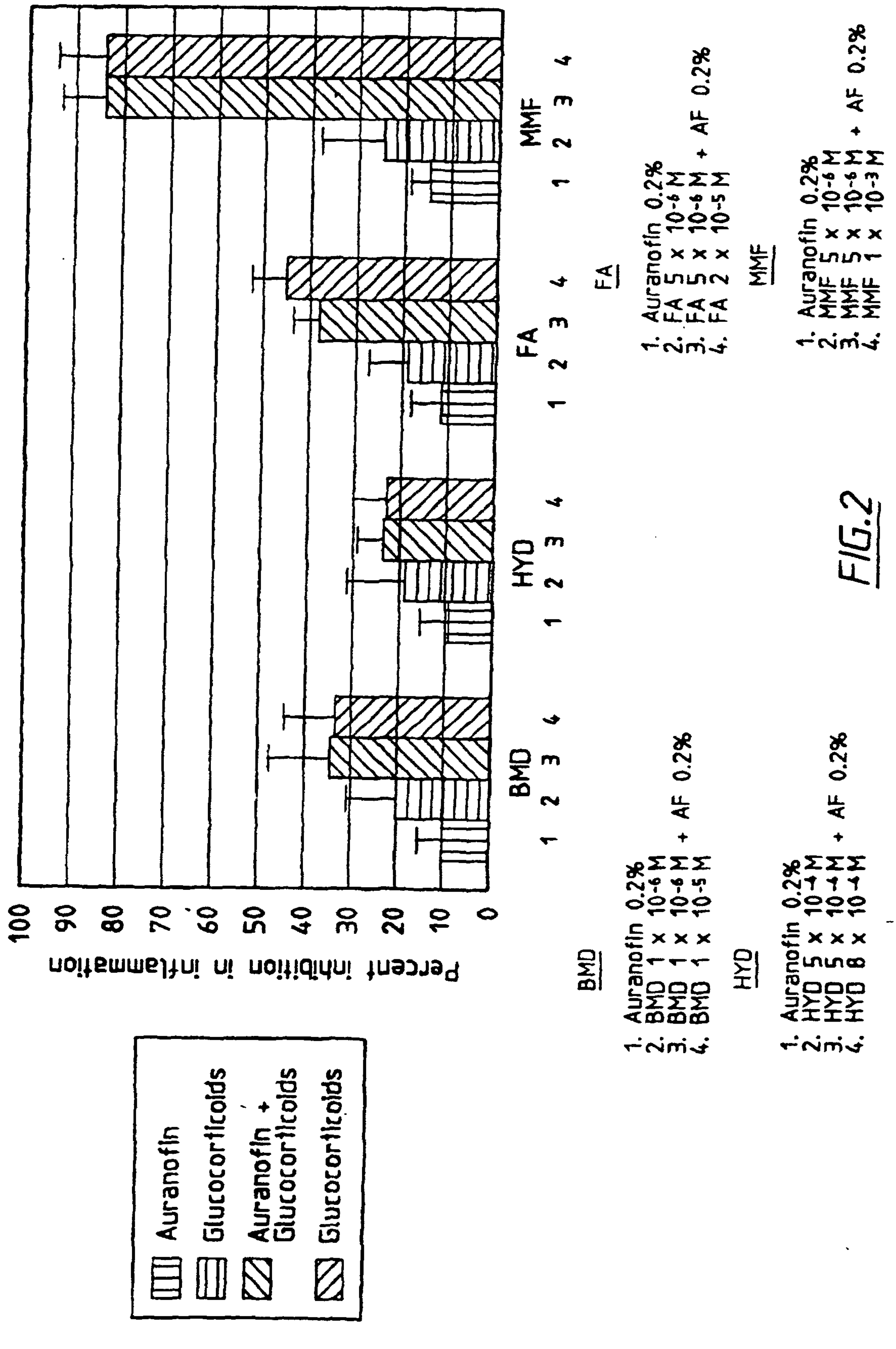
Auranofin
Glucocorticoids
Auranofin + Glucocorticoids
Glucocorticoids
Percent inhibition in inflammation
100
90
80
70
60
50
40
30
20
10
0
1 2 3 4
BMD
1 2 3 4
HYD
1 2 3 4
FA
1 2 3 4
MMF
BMD
1. Auranofin 0.2%
2. BMD 1 x 10-6 M
3. BMD 1 x 10-6 M + AF 0.2%
4. BMD 1 x 10-5 M
HYD
1. Auranofin 0.2%
2. HYD 5 x 10-4 M
3. HYD 5 x 10-4 M + AF 0.2%
4. HYD 8 x 10-4 M
FA
1. Auranofin 0.2%
2. FA 5 x 10-6 M
3. FA 5 x 10-6 M + AF 0.2%
4. FA 2 x 10-5 M
MMF
1. Auranofin 0.2%
2. MMF 5 x 10-6 M
3. MMF 5 x 10-6 M + AF 0.2%
4. MMF 1 x 10-3 M

FIG.2